# EUROPÄISCHE PATENTSCHRIFT

(11) **EP 1 131 042 B1**
(45) Veröffentlichungstag und Bekanntmachung des Hinweises auf die Patenterteilung: **21.01.2004**
(21) Anmeldenummer: 99972112.9
(22) Anmeldetag: 06.11.1999
(51) Int. Cl.: A61K 7/13

(54) **FÄRBEMITTEL MIT ÜBERGANGSMETALLKOMPLEXEN**
COLORANTS WITH TRANSITION METAL COMPLEXES
COLORANT CONTENANT DES COMPLEXES A METAUX DE TRANSITION

(30) Priorität: 17.11.1998 DE 19852972
(43) Veröffentlichungstag der Anmeldung: 12.09.2001
(73) Patentinhaber: Henkel Kommanditgesellschaft auf Aktien, 40589 Düsseldorf (DE)
(72) Erfinder: MAYER, Bernd, D-40593 Düsseldorf (DE); HELLER, Melita, D-40591 Düsseldorf (DE); BLUM, Helmut, D-40595 Düsseldorf (DE); HÖFFKES, Horst, D-40595 Düsseldorf (DE)
(86) Internationale Anmeldenummer: PCT/EP1999/008527
(87) Internationale Veröffentlichungsnummer: WO 2000/029036

(56) Entgegenhaltungen:
- EP-A- 0 437 875
- EP-A- 0 507 448
- EP-A- 0 621 029
- WO-A-96/03643
- WO-A-98/01418
- FR-A- 1 439 307
- US-A- 3 429 646
- US-A- 5 708 022
- US-A- 5 715 845
- YAMAMOTO,S. ET AL.: "Bleach activation catalyst and bleach compositions containing the same showing good bleaching efficiency even at low temperatures" STN INTERNATIONAL, FILE CAPLUS, AN129:55804, XP002136263 & JP 10 140193 A (LION CORP., JAPAN) 26. Mai 1998 (1998-05-26)
- KRAUSE, H.W.: "Catalytic decomposition of hydrogen peroxide by iron chelates" STN INTERNATIONAL, FILE CAPLUS, AN 70:100078, XP002136264 & KATAL. REAKTS. ZHIDK. FAZE, T. VSES. KONF.,2ND, ALMA-ATA, KAZ. SSR.,1966, Seiten 491-495,
- PATENT ABSTRACTS OF JAPAN vol. 1995, no. 08, 29. September 1995 (1995-09-29) & JP 07 126176 A (HIROSHI SAKURAI;OTHERS: 01), 16. Mai 1995 (1995-05-16) & DATABASE WPI Derwent Publications Ltd., London, GB; AN 1996-303518

## Beschreibung

Die vorliegende Erfindung betrifft Mittel zum Färben keratinischer Fasern, die speziellen Übergangsmetallkomplexe als katalytisch wirksame Aktivatoren enthalten sowie deren Verwendung.

Für das Färben von keratinhaltigen Fasern, z.B. Wolle, Pelzen und insbesondere menschlichen Haaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung.

Direktziehende Farbstoffe werden unter schonenden Bedingungen appliziert. Ihr Nachteil liegt jedoch darin, daß die Färbungen häufig nur über unzureichende Echtheitseigenschaften verfügen. Mit Oxidationsfarbstoffen lassen sich zwar intensive Färbungen mit guten Echtheitseigenschaften erzielen, die Entwicklung der Farbe geschieht jedoch in der Regel unter dem Einfluß von H₂O₂ oder H₂O₂-Addukten, was Schädigungen der Faser zur Folge haben kann.

Auch Oxidationsfärbemittel mit Luftsauerstoff als sehr mildem Oxidationsmittel wurden bereits vorgeschlagen; im allgemeinen laufen die Oxidationen mit Luftsauerstoff jedoch nicht vollständig ab. Insbesondere in Oxidationshaarfärbemitteln, die in der Regel in einem cremeartigen kosmetischen Träger appliziert werden, findet keine zufriedenstellende Diffusion des Luftsauerstoff zu den Farbstoffvorprodukten statt. Weiterhin wurde, beispielsweise in der EP-B 1-0 548 620, vorgeschlagen, den Gehalt an Wasserstoffperoxid in den Oxidationsfärbemitteln auf 1 Gew.-% und weniger zu begrenzen, dem Mittel aber gleichzeitig zur Aktivierung spezielle Enzyme, Peroxidasen, zuzufügen. Diese Ansätze haben aber bisher nicht vollständig überzeugt.

Es besteht daher nach wie vor die Aufgabe, Oxidationsfärbemittel zu entwickeln, die aufgrund ihres geringeren Gehaltes an Oxidationsmitteln schonendere Färbungen ermöglichen, ohne das Färbeergebnis negativ zu beeinflussen. Weiterhin ist es in hohem Maße wünschenswert, den pH-Wert der Färbemittel auf einen Wert um den Neutralpunkt zu senken, um möglichen Schädigungen der Faser aufgrund der üblicherweise starken Alkalinität der Färbemittel vorzubeugen.

Es wurde nunmehr überraschenderweise gefunden, daß durch Verwendung spezieller Übergangsmetallkomplexe als Oxidationskatalysatoren in Mitteln zur Färbung keratinischer Fasern
- die Menge des Oxidationsmittels stark reduziert werden kann und/oder
- die Ausfärbung im neutralen pH-Bereich erfolgen kann.

Der Einsatz von Übergangsmetallkomplexen im Zusammenhang mit einer oxidativen Haarfärbung ist prinzipiell bekannt. So wurde in der europäischen Patentanmeldung EP-A2-507 448 vorgeschlagen, die Haare vor dem eigentlichen Färbeprozeß mit einem Übergangsmetallkomplex, enthaltend die Liganden 2,2'-Dipyridyl oder o-Phenanthrolin, zu behandeln, um das Haar gegen einen oxidativen Abbau des Cysteins zu stabilisieren. In der deutschen Patentschrift DE-C1-195 34 214 wurde weiterhin vorgeschlagen, Übergangsmetallkomplexe, insbesondere Kupferkomplexe als Oxidationskatalysatoren in Haarfärbemitteln einzusetzen, die als Entwicklerkomponenten Derivate des 4-(2,5-Diaminophenoxymethyl)-1,3-dioxolans enthalten. Schließlich wurde in der deutschen Anmeldung P 197 57 510.2 vorgeschlagen, Übergangsmetallkomplexe mit Liganden vom Salen-Typ als Oxidationskatalysatoren einzusetzen. Dennoch bestand weiterhin Bedarf an neuartigen Oxidationskatalysatoren für die oxidative Haarfärbung.

Ein erster Gegenstand der vorliegenden Erfindung sind daher Mittel zur Färbung keratinischer Fasern, die mindestens ein Farbstoffvorprodukt enthalten sowie 0,0001 bis 1,0 Gew.-% eines Übergangsmetallkomplexes mit
- einem oder mehreren Übergangsmetallkationen, ausgewählt aus Eisen, Kobalt, Mangan, Molybdän, Ruthenium und/oder Vanadium sowie
- mindestens einem zweizähnigen oder mehrzähnigen Liganden, bei dem mindestens eine Koordinationsstelle durch einen stickstoffhaltigen Heterocyclus gebildet wird.
mit der Maßgabe, daß der Komplex mindestens ein Rutheniumkation enthält.

Unter stickstoffhaltigen Heterocyclen sind entsprechend der Definition von Römpp Chemie Lexikon, 10. Auflage, Bd. 3, S. 1739, Georg Thieme Verlag cyclische organische Verbindungen zu verstehen, deren Ringstrukturen neben Kohlenstoffatomen noch mindestens ein Stickstoffatom und gegebenenfalls weitere Heteroatome enthalten.

Zweizähnige beziehungsweise mehrzähnige Liganden sind erfindungsgemäß Moleküle, die zwei oder mehrere Elektronendonatorzentren aufweisen.

Die Übergangsmetallkationen liegen in den erfindungsgemäßen Mitteln bevorzugt in ihrer höchsten stabilen Oxidationsstufe vor.

Komplexe mit Eisen und/oder Rutheniumkationen haben sich als erfindungsgemäß besonders geeignet erwiesen.

Als Liganden in den erfindungsgemäß anwendbaren Übergangsmetallkomplexen kommen prinzipiell alle Substanzen in Betracht, die neben einer weiteren Koordinationsstelle mindestens einen stickstoffhaltigen Heterocyclus wie beispielsweise Pyridin, Pyridazin, Pyrimidin, Pyrazin, Imidazol, Pyrazol und/oder Triazol enthalten. Solche sind beispielsweise 2 2'-Bispyridylamin, 1,4,7-Triazacyclononan, 1,4,7-2,2'-Bispyridylamin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, NNN',N'-Tetrakis-(2-benzimidazolylmethyl)-2-hydroxy-1,3-diaminopropan, 2,6-Bis-(bis-(2-pyridylmethyl)-aminomethyl)-4-methylphenol, 1,3-Bis-(bis-(2-benzimidazolylmethyl)aminomethyl)-benzol, 1,4,8,11-Tetraazacyclotetradecan, 1,8-Diazabicyclo[5.4.0]undec-7-en(1,5-5), Pyridin-2,6-dicarbonsäure, 2,2':6',2"-Terpyridin, 1,10-Phenanthrolin, Tris(2-pyridylmethyl)amin, Tris(2-pyridylethyl)amin sowie N-Carboxy-methyl-N-(2-pyridylmethyl)glycin und gegebenenfalls substituierte Porphine und Porphyrine.

Neben den oben aufgezählten Liganden können erfindungsgemäß auch deren substituierte Derivate eingesetzt werden. Als Substituenten sind C₁- bis C₄-Alkylgruppen, Hydroxygruppen, Carboxylgruppen, Nitrogruppen sowie Sulfonsäurefunktionen besonders bevorzugt.

Die folgenden Liganden haben sich als erfindungsgemäß besonders geeignet erwiesen:
- 1,4, 8,11 -Tetraazacyclotetradecan
- 1,8-Diazabicyclo[5.4.0]undec-7-en(1,5-5)
- Pyridin-2,6-dicarbonsäure
- 2,2':6',2"-Terpyridin
- 1,10-Phenanthrolin
- Tris(2-pyridylmethyl)amin
- Tris(2-pyridylethyl)amin sowie
- N-Carboxymethyl-N-(2-pyridylmethyl)glycin.

Ganz besonders geeignet sind 1,10-Phenanthrolin, Tris-(2-pyridylmethyl)-amin, 1,4,8,11-Tetraazacyclotetradecan sowie 2,2':6',2"-Terpyridin.

Es kann erfindungsgemäß bevorzugt sein, Liganden mit einem aromatischen, stickstoffhaltigen Heterocylcus zu verwenden.

Neben den erfindungswesentlichen mehrzähnigen Liganden können die Übergangsmetallkomplexe noch weitere Liganden aufweisen. Zu den anorganischen Neutral-liganden gehören insbesondere Ammoniak und Wasser. Insbesondere bei den Co(III)-Komplexen, in denen das Zentralatom normalerweise mit der Koordinationszahl 6 vorliegt, ist die Anwesenheit von mindestens 1 Ammoniak-Liganden bevorzugt.

Falls nicht sämtliche weiteren Koordinationsstellen des Übergangsmetallzentralatoms durch Neutralliganden besetzt sind, enthält ein gemäß der Erfindung zu verwendender Komplex weitere, vorzugsweise anionische und unter diesen insbesondere ein- oder zweizähnige Liganden. Zu diesen gehören insbesondere die Halogenide wie Fluorid, Chlorid, Bromid und Iodid, und die (NO₂)⁻-Gruppe. Unter einer (NO₂)⁻-Gruppe soll im Rahmen dieser Anmeldung sowohl ein Nitro-Ligand, der über das Stickstoffatom an das Übergangsmetall gebunden ist, als auch ein Nitrito-Ligand, der über ein Sauerstoffatom an das Übergangsmetall gebunden ist, verstanden werden. Die (NO₂)⁻-Gruppe kann an ein Übergangsmetall auch chelatbildend gebunden sein oder sie kann zwei Übergangsmetallatome asymmetrisch oder µ¹-O-verbrücken.

Außer den genannten Liganden können die im Aktivatorsystem gemäß der Erfindung zu verwendenden Übergangsmetallkomplexe noch weitere Liganden, insbesondere ein- oder mehrwertige Anionliganden, tragen. In Frage kommen beispielsweise Nitrat, Actetat, Trifluoracetat, Formiat, Carbonat, Citrat, Perchlorat sowie komplexe Anionen wie Hexafluorophosphat. Die Anionliganden sind für den Ladungsausgleich zwischen Übergangsmetall-Zentralatom und dem Ligandensystem notwendig. Auch die Anwesenheit von Oxo-Liganden, Peroxo-Liganden und Imino-Liganden ist möglich. Insbesondere derartige Liganden können auch verbrückend wirken, so daß mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome gleich oder ungleich sind und unterschiedliche Oxidationsstufen aufweisen, ist möglich.

Falls Anionliganden fehlen oder die Anwesenheit von Anionliganden nicht zum vollständigen Ladungsausgleich im Komplex führt, können in den gemäß der Erfindung zu verwendenden Übergangsmetallkomplex-Verbindungen anionische Gegenionen anwesend sein, die den kationischen Übergangsmetall-Komplex neutralisieren. Zu diesen anionischen Gegenionen gehören insbesondere Nitrat, Hydroxid, Hexafluorophosphat, Tetrafluoroborat, Sulfat, Chlorat, Perchlorat, die Halogenide wie Chlorid oder die Anionen von Carbonsäuren wie Formiat, Acetat, Benzoat oder Citrat.

In einer ersten Ausführungsform der vorliegenden Erfindung kann das Farbstoffvorprodukt ein Oxidationsfarbstoffvorprodukt vom Typ Entwickler sein. Es können auch mehrere Entwickler gemeinsam in den erfindungsgemäßen Mitteln eingesetzt werden.

Entwicklersubstanzen sind üblicherweise aromatische oder heterocyclische Ringsysteme, die durch zwei in ortho- oder para-Stellung zueinander stehende reaktive Gruppen, i.a. Hydroxy- oder Aminogruppen, gekennzeichnet sind. Solche Verbindungen sind beispielsweise primäre aromatische Amine mit einer weiteren, in para- oder ortho-Position befindlichen freien oder substituierten Hydroxy- oder Aminogruppe, ferner Diaminopyridinderivate, heterocyclische Hydrazonderivate oder 4-Amino-pyrazolonderivate.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Aminomethyl-4-aminophenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin sowie 4-Hydroxy-2,5,6-triaminopyrimidin.

Zur Nuancierung der erzielbaren Farbtöne können die erfindungsgemäßen Mittel weiterhin noch eine oder mehrere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-aminophenol, 2-Methylresorcin, 5-Methylresorcin, 2,5-Dimethylresorcin sowie 2,6-Dihydroxy-3,4-diaminopyridin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer zweiten bevorzugten Ausftihrungsform des Gegenstandes der vorliegenden Erfindung kann das Farbstoffvorprodukt ein Derivat des Indolins der Formel (Ia) sein, 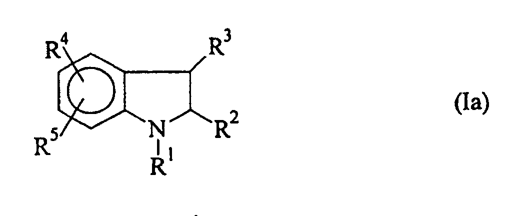 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure.

In einer dritten bevorzugten Ausführungsform dieses Gegenstandes der vorliegenden Erfindung kann das Farbstoffvorprodukt ein Derivat des Indols der Formel (Ib) sein, 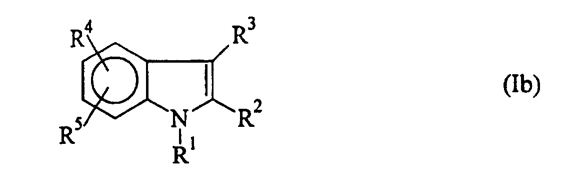 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure.

Bevorzugte Stoffe der Formel (1a) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (1b) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Ganz besonders bevorzugt sind 5,6-Dihydroxyindol sowie 5,6-Dihydroxyindolin.

In einer ersten bevorzugten Variante der oben beschriebenen Ausführungsformen werden die Mittel derart formuliert, daß sie als Farbstoffvorprodukte nur Indol- und/oder Indolinderivate der Formeln (1a) und (1b) enthalten und frei sind von üblichen Oxidationsfarbstoffvorprodukten vom Entwickler- bzw. Kupplertyp.

In einer zweiten bevorzugten Variante der oben beschriebenen Ausführungsformen können die erfindungsgemäßen Mittel neben den Indol- und/oder Indolinderivaten der Formeln (1a) und (1b) auch noch übliche Oxidationsfarbstoffvorprodukte vom Entwickler- bzw. Kupplertyp enthalten.

Es kann erfindungsgemäß besonders bevorzugt sein, die Indol- und/oder die Indolinderivate der Formeln (1a) und (1b) in Kombination mit einer oder mehrere Kupplerkomponenten in Haarfärbemitteln einzusetzen. Beispielhaft sei an dieser Stelle ausdrücklich auf die oben genannten Kupplerkomponenten verwiesen.

Weiterhin kann es erfindungsgemäß bevorzugt sein, die Indol- und/oder Indolinderivate der Formel (1a) und (1b) in Kombination mit mindestens einer Aminosäure oder einem Oligopeptid in Haarfärbemitteln einzusetzen. Es kann erfindungsgemäß weiterhin bevorzugt sein, wenn die Aminosäure eine α-Aminosäure ist. Ganz besonders bevorzugte α-Aminosäuren sind Arginin, Omithin, Lysin und Histidin.

In einer bevorzugten Ausführungsform enthalten die erfindungsgemäßen Haarfärbemittel zur weiteren Modifizierung der Farbnuancen neben den Farbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2. HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1. Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbemitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europälsche Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Färbungen von besonderer Farbtiefe können erreicht werden, wenn die Mittel neben den Farbstoffen und/oder Farbstoffvorprodükten zusätzlich noch ein Öl des Wiesenschaumkrauts (INCI-Bezeichnung: Meadowfoam Seed Oil) enthalten.

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen.

Die erfindungsgemäßen Färbemittel können weiterhin alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Färbemittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis. 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure. Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂- bis C₂₂-Fettsäuremono- und -diester von Anlagerungsprodukten von 1 bis 30 Mol Ethylenoxid an Glycerin,
- C₈- bis C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethyl-ammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈- bis C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂bis C₁₈-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaterniurn-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyl-dialkoyloxyalkyl-ammonium-methosulfate sowie die unter dem Warenzeichen Dehyquart® vertriebenen Produkte.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Je nach Art des Mittels und des Tensidtyps sind die Tenside in den erfindungsgemäßen Mitteln üblicherweise in Mengen von insgesamt 0,5 bis 30 Gew.-%, bezogen auf das gesamte Mittel, enthalten.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat® 755 im Handel erhältlich.
- Vinylpyrrolidon-Methoimidazoliniumchlorid-Copolymere, wie sie unter der Bezeichnung Luviquat® angeboten werden:
- quatemierter Polyvinylalkohol
sowie die unter den Bezeichnungen
- Polyquatemium 2,
- Polyquatemium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen.

Als konditionierende Wirkstoffe weiterhin geeignet sind Silikonöle, insbesondere Dialkylund Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Ebenfalls einsetzbar als konditionierende Wirkstoffe sind Paraffinöle sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Schließlich enthalten die erfindungsgemäßen Färbemittel bevorzugt noch einen Fettstoff.

Bevorzugte Fettstoffe sind lineare und verzweigte, gesättigte und ungesättigte Fettalkohole oder natürliche Fettalkoholgemisch mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren. Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.-Butylacrylamid-Terpolymere,
- symmetrische und unsymmetrische, lineare und verzweigte Dialkylether mit insgesamt zwischen 12 bis 36 C-Atomen, insbesondere 12 bis 24 C-Atomen, beispielsweise Din-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Antischuppenwirkstoffe wie Piroctone Olamine, Zink Omadine und Climbazol,
- tierische und pflanzliche Proteinhydrolysate, insbesondere Elastin-, Kollagen-, Keratin-, Milcheiweiß-, Sojaprotein-, Mandelprotein- und Weizenproteinhydrolysate, sowie deren Fettsäurekondensationsprodukte und quaternierte Derivate,
- Vitamine und Vitaminvorstufen wie Panthenol, dessen Derivate und Biotin,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- Weitere Wirkstoffe wie Ceramide, Allantoin, Pyrrolidoncarbonsäuren, und Bisabolol,
- Lichtschutzmittel,
- Entschäumer wie Silikone,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Cholesterin,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Konsistenzgeber wie Zuckerester, Polyolester oder Polyolalkylether,
- Strukturanten wie Maleinsäure, Mono-, Di- und Oligosaccharide,
- Fette und Wachse, wie Walrat, Bienenwachs, Montanwachs und Paraffine,
- Fettsäurealkanolamide,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- Treibmittel wie Propan-Butan-Gemische, N₂O Dimethylether, CO₂, N₂ und Luft

Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am menschlichen Haar gewünscht ist. Als Oxidationsmittel kommen insbesondere Wasserstoffperoxid oder dessen Anlagerungsprodukte an Harnstoff Melamin oder Natriumborat in Frage. Weiterhin ist es möglich, die Oxidation mit Hilfe von Enzymen durchzuführen. Dabei können die Enzyme sowohl zur Erzeugung von oxidierenden Perverbindungen eingesetzt werden, als auch zu Verstärkung der Wirkung einer geringen Menge vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Die Übergangsmetallkomplexe können erfindungsgemäß sowohl in einer gemeinsamen Zubereitung mit den Farbstoffvorprodukten als auch separat konfektioniert sein.

In einer bevorzugten Ausführungsform der erfindungsgemäßen Lehre werden die separat konfektionierten Übergangsmetallkomplexe in einem geeigneten Lösemittel, beispielsweise in Wasser, Ethanol oder Aceton, gelöst und unmittelbar vor dem Färben der Haare mit der Oxidationsmittelzubereitung verrührt. Diese Zubereitung wird anschließend mit einer Zubereitung, enthaltend die Farbstoffvorprodukte, vermischt. Das dabei entstehende gebrauchsfertige Haarfärbepräparat sollte bevorzugt einen pH-Wert im Bereich von 6 bis 10 aufweisen. Besonders bevorzugt ist ein pH-Wert von 6,5 bis 8. Die Anwendungstemperaturen können in einem Bereich zwischen 15 und 40°C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbe-shampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Ein zweiter Gegenstand der vorliegenden Erfindung ist die Verwendung der obengenannten Mittel zur Färbung keratinischer Fasern.

Durch die erfindungsgemäße Verwendung der Übergangsmetall-Komplexe ist es möglich
- die üblicherweise nur unter alkalischen Bedingungen erhaltenen Färbeleistungen auch im neutralen pH-Bereich zu erzielen oder
- eine schonendere Färbebehandlung dadurch zu erzielen, daß im alkalischen Bereich die angestrebte Färbeleistung bereits mit deutlich, d.h. bis zu 75 %, niedrigeren Konzentrationen an Oxidationsmittel erreicht wird. So kann beispielsweise die Wasserstoffperoxidkonzentration in einer Anwendungszubereitung von ca. 3 Gew.-% auf ca. 1,0 Gew.-%-gesenkt werden.

### Beispiele

Es wurde eine Färbecreme folgender Zusammensetzung hergestellt (alle Angaben sind, soweit nicht anders vermerkt, in g):

| **Teilmischung A** | |
|---|---|
| Hydrenöl® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Cetylstearylalkohol mit ca. 20 EO-Einheiten (INCI-Bezeichnung: Ceteareth-20) (HENKEL) | |
| ⁴ Laurylethersulfat, Natriumsalz (ca. 27,5% Aktivsubstanz; INCI-Bezeichnung: Sodium Laureth Sulfate) (HENKEL) | |
| ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol D, Lorol und Eumulgin B2 wurden bei 80°C aufgeschmolzen, mit dem 80°C heißem Wasser, enthaltend Texapon NSO und Dehyton K, vermischt und unter starkem Rühren emulgiert. Danach wurde die Emulsion unter kontinuierlichem Rühren abgekühlt.

| **Teilmischung B** | |
|---|---|
| Natriumsulfit | 1,00g |
| Ammoniumsulfat | 1,00g |
| 5-Amino-2-methylphenol | 0,003mol |
| 4-Amino-2-aminomethylphenol-hydrochlorid | 0,003mol |
| Ammoniak (25%ige Lösung) | ad pH 8,7 |
| Wasser | 10,00g |

Die Farbstoffvorprodukte wurden in dem 50°C heißem Wasser unter Zugabe von Natriumsulfit, Ammoniumsulfat und Ammoniak gelöst.
Die Farbstoffvorproduktlösung (Teilmischung B) wurde zur Emulsion (Teilmischung A) gegeben, mit einer Ammoniaklösung auf den gewünschten pH-Wert eingestellt und mit Wasser auf 100 Gewichtsteile aufgefüllt. Es wurde bis zum Erreichen der Raumtemperatur weitergerührt. Die Färbecreme wies einen pH-Wert von 8,7 auf.

Als Entwickler wurden 10 ml einer 3 Gew.-%igen wäßrigen Wasserstoffperoxidlösung verwendet. Der Übergangsmetall-Komplex wurde in einigen Tropfen Wasser vorgelöst und zu der Entwicklerlösung gegeben.
Sodann wurden Entwicklerlösung und 12,5g Färbecreme vereinigt; die anwendungsbereite Färbemischung hatte jeweils einen pH-Wert von 7,0 - 7,5. Anschließend wurde die Anwendungszubereitung auf eine Haarsträhne der Sorte "Kerling Naturweiß" (1,5 g Anwendungszubereitung pro g Haar), gegeben, dort 30 Minuten belassen und dann mit Wasser ausgespült.
Zunächst wurde eine Anwendungszubereitung mit einer Konzentration von 222ppm Übergangsmetallkationen in der Fertigmischung hergestellt. Es wurden weiterhin Ausfärbungen mit einer Konzentration von 111 ppm Übergangsmetallkationen, 55,5ppm Übergangsmetallkationen, 28ppm Übergangsmetallkationen sowie 14ppm Übergangsmetallkationen, jeweils bezogen auf die gesamte Anwendungszubereitung, durchgeführt.

| **Metall** | **Ligand** | **Gegenion** | **Bewertung** |
|---|---|---|---|
| *Fe³⁺ | 2,2':6',2"-Terpyridin | ClO₄⁻ | ++ |
| *Fe³⁺ | 1,10-Phenanthrolin | ClO₄⁻ | +++ |
| Ru³⁺ | 1,10-Phenanthrolin | ClO₄⁻ | ++ |
| Ru³⁺ | Tris-(2-pyridylmethyl)-amin | ClO₄⁻ | +++ |
| Ru³⁺ | Tetraazacyclotetradecan | ClO₄⁻ | +++ |
| Ru³⁺ | 2,2':6',2"-Terpyridin | ClO₄⁻ | +++ |

| | | | |
|---|---|---|---|
| ^{*} Beispiel außerhalb des Schutzbereichs der Ansprüche Bewertungsmaßstab - keine Färbung bei Einsatz von mindestens 111 ppm Übergangsmetallkationen + rötlich-braune Färbung bei Einsatz von mindestens 111 ppm Übergangsmetallkationen ++ rötlich-braune Färbung bei Einsatz von mindestens 28ppm Übergangsmetallkationen +++ rötlich-braune Färbung bei Einsatz von mindestens 14ppm Übergangsmetallkationen | | | |

Ein entsprechender Versuch ohne Zugabe des Übergangsmetallkomplexes ergab keine Färbung.

## Patentansprüche

1. Mittel zum Färben keratinischer Fasern, insbesondere menschlicher Haare, enthaltend mindestens ein Farbstoffvorprodukt vom Typ der Entwickler und/oder der Indol- und/oder Indolinderivate, **dadurch gekennzeichnet, daß** es 0,0001 bis 1,0 Gew.-% eines Übergangsmetallkomplexes enthält, der
- ein oder mehrere Übergangsmetallkationen, ausgewählt aus Eisen, Kobalt, Mangan, Molybdän, Ruthenium und/oder Vanadium sowie
- mindestens einen zweizähnigen oder mehrzähnigen Liganden, bei dem mindestens eine Koordinationsstelle durch einen stickstoffhaltigen Heterocyclus gebildet wird,
enthält, mit der Maßgabe, daß der Komplex mindestens ein Rutheniumkation enthält.

2. Mittel nach Anspruch 1, **dadurch gekennzeichnet, daß** der Übergangsmetallkomplex mindestens einen Liganden enthält, der aus einer Gruppe stammt, die gebildet wird von
- 1,4,8,11-Tetraazacyclotetradecan
- 1,8-Diazabicyclo[5.4.0]undec-7-en(1,5-5)
- Pyridin-2,6-dicarbonsäure
- 2,2':6',2"-Terpyridin
- 1,10-Phenanthrolin
- Tris(2-pyridylmethyl)amin
- Tris(2-pyridylethyl)amin sowie
- N-Carboxymethyl-N-(2-pyridylmethyl)glycin.

3. Mittel nach einem der Ansprüche 1 bis 2, **dadurch gekennzeichnet, daß** der stickstoffhaltige Heterocyclus des Liganden aromatisch ist.

4. Mittel nach einem der Ansprüche 1 bis 3, **dadurch gekennzeichnet, daß** es einen pH-Wert von 6,5 bis 8 aufweist.

5. Verwendung eines der Mittel der Ansprüche 1 bis 4 zur oxidativen Färbung keratinischer Fasern.

## Claims

1. A composition for coloring keratin fibers, more particularly human hair, containing at least one dye precursor of the primary intermediate and/or indole derivative and/or indoline derivative type, **characterized in that** it contains 0.0001 to 1.0% by weight of a transition metal complex containing
- one or more transition metal cations selected from iron, cobalt, manganese, molybdenum, ruthenium and/or vanadium and
- at least one bidentate or polydentate ligand in which at least one coordination site is formed by a nitrogen-containing heterocycle,
with the proviso that the complex contains at least one ruthenium cation.

2. A composition as claimed in claim 1, **characterized in that** the transition metal complex contains at least one ligand from a group consisting of
- 1,4,8,11-tetraazacyctotetradecane
- 1,8-diazabicyclo[5.4.0]undec-7-ene (1,5-5)
- pyridine-2,6-dicarboxylic acid
- 2,2':6',2"-terpyridine
- 1,10-phenanthroline
- tris(2-pyridylmethyl)amine
- tris(2-pyridylethyl)amine and
- N-carboxymethyl-N-(2-pyridylmethyl)glycine.

3. A composition as claimed in claim 1 or 2, **characterized in that** the nitrogen-containing heterocycle of the ligand is aromatic.

4. A composition as claimed in any of claims 1 to 3, **characterized in that** it has a pH value of 6.5 to 8.

5. The use of one of the compositions claimed in claims 1 to 4 for the oxidative coloring of keratin fibers.

## Revendications

1. Agent de coloration de fibres kératiniques, en particulier de cheveux humains, contenant au moins un précurseur de colorant du type des révélateurs et/ou des dérivés d'indole et/ou d'indoline, **caractérisé en ce qu'**il contient de 0,0001 à 1,0% en poids d'un complexe de métal de transition, lequel contient
- un ou plusieurs cations de métaux de transition, choisis parmi le fer, le cobalt, le manganèse, le molybdène, le ruthénium et/ou le vanadium ainsi que
- au moins un ligand bidenté ou polydenté, dans lequel au moins un endroit de coordination est formé par un hétérocycle azoté, sous réserve que le complexe contient au moins un cation ruthénium.

2. Agent selon la revendication 1, **caractérisé en ce que** le complexe de métal de transition contient au moins un ligand provenant d'un groupe formé par
- le 1,4, 8,11-tétraazacyclotétradécane,
- le 1,8-diazabicyclo[5.4.0]undéc-7-ène(1,5-5,),
- l'acide pyridine-2,6-dicarboxylique,
- la 2,2' ;6',2"-terpyridine,
- la 1,10-phénanthroline,
- la tris(2-pyridylméthyl)amine,
- la tris(2-pyridyléthyl)amine ainsi que
- la N-carboxyméthyl-N-(2-pyridylméthyl)glycine.

3. Agent selon l'une des revendications 1 à 2, **caractérisé en ce que** l'hétérocycle azoté du ligand est aromatique.

4. Agent selon l'une des revendications 1 à 3, **caractérisé en ce qu'**il présente un pH de 6,5 à 8.

5. Utilisation d'un des agents des revendications 1 à 4 pour la coloration par oxydation de fibres kératiniques.
